# EUROPEAN PATENT APPLICATION

(11) **EP 2 364 745 A1**
(43) Date of publication of application: **14.09.2011**
(21) Application number: 11157997.5
(22) Date of filing: 14.03.2011
(51) Int. Cl.: A61M 21/00

(54) **Vibrational delta and theta brain wave induction apparatus and method for stimulation of sleep**

(30) Priority: 12.03.2010 US 723363
(71) Applicant: Sunnen, Gerard V., New York NY 10016 (US)
(72) Inventor: Sunnen, Gerard V., New York NY 10016 (US)
(74) Representative: Vinazzer, Edith

(57) **Abstract**

An apparatus and method for generating sleep-inducing stimuli, including a programmable controller operable for generating a sleep-inducing rhythm; and a transducer unit containing at least one transducer connected to the controller for receiving the sleep-inducing rhythm and generating and applying the sleep-inducing stimuli to a user in accordance with the rhythm. The stimuli may be in the form of vibration, warmth, light, sound, and/or electrical current. The stimuli are adapted to induce alpha, theta, and/or delta brain waves to induce sleep. The vibration may be provided by a motor unit generating and applying at least one of horizontal, vertical, circular, and smooth repetitive 3-dimensional vibration as stimuli to prompt the production of slow brain waves of said user. Preferably the motor unit generates at least two of horizontal, vertical, circular, and smooth repetitive 3-dimensional vibrational stimuli.

## Description

### CROSS-REFERENCE TO RELATED APPLICATIONS

The present application claims priority of U.S. Serial No. 12/723,363 filed March 12, 2010, and is a continuation-in-part of U.S. Serial No. 11/075,075 filed March 8, 2005, both incorporated herein by reference.

### BACKGROUND AND SUMMARY OF THE INVENTION

Sleep disorders are increasing in their incidence and prevalence in the general population and pose enormous public health issues. An effective therapy for the most common sleep disorders could bring massive public health benefits in terms of physical health, psychological well-being, and productivity. Indeed, sleep disorders have been strongly associated with depression and anxiety.

The normal sleep cycle is divided into two main phases each embodying characteristic physiological constellations.

Sleep centers in the nervous system regulate the rhythm of circadian sleep/wake cycles. The suprachiasmatic (SNC) nucleus of the anterior hypothalamus receives impulses from retinal nerves and other special senses. The SNC projects nerve fibers into the hypothalamus which, in turn, has influence over locomotor activity, food and water intake, body temperature, and hormone levels. It is apparent, therefore, that sleep functions actively weave into the spectrum of basic bodily functions.

In one of these phases, a salient feature is the emergence of rapid eye movement (REM). This phase is called REM sleep. In the other phase, eye movements are relatively absent. This phase is called non-REM, or NREM sleep. During the course of the night, there are several periods of alternating REM and NREM sleep phases.

REM periods are associated with, in addition to eye motions, EEG rhythms found in the waking state such as alpha waves (8 to 12 cycles per second), the inhibition of muscle activity, the engagement of the autonomic nervous system as expressed in blood pressure and heart rate fluctuations, and with dreaming. 20% to 25% of sleep time is devoted to REM sleep. In normal sleep, an initial NREM phase of approximately 70 to 100 minutes duration is followed by the first REM period. Depending upon total sleep time, this cycle is repeated 4 to 6 times during the night.

NREM cycles do not show the characteristic horizontal and vertical eye motions found in REM sleep. In the beginning of the nightly sleep cycle, alpha waves begin to give way to low-voltage, theta, 4 to 7 cycles per second brain waves. This is identified as stage 1 sleep.

Stage 2 sleep usually occurs less than a minute later, but may be delayed for several minutes. 12 to 14 cycle spindle tracings appear with occasional slow triphasic waves, known as K complexes.

Soon thereafter, cycles ranging from 4 to 0.5 cycles per second appear. These are known as delta waves. When occupying less than 50% of the tracing, this is designated as stage 3 sleep. When delta waves account for more than 50% of the EEG tracing, stage 4 is achieved. Taken together, stages 3 and 4 are known as delta sleep or slow wave sleep (SWS).

In the transition from full wakefulness to somnolence, drowsiness, slumber, and finally sleep, waking EEG patterns give way to stages 1 through 4 NREM sleep. Delta sleep provides the most recuperative, highly quality sleep.

Individuals afflicted with initial insomnia have difficulties transitioning from the waking state to stages 1 and 2, and on to stages 3 and 4.

The disclosed method and apparatus aim to assist in the therapy of the most common manifestation of sleep dysfunction, namely the difficulty in falling asleep, so-called initial insomnia.

A way of doing so would be to prompt sleep-generating brain waves in brain sleep centers by stimulating sensory organs such as the skin, the visual senses, and the auditory senses with delta rhythms.

The delta wave prompting, importantly, is preferably individualized. Indeed, individuals show important variations in their preferences for the frequencies and the properties of the vibrations, and their translation into sound and color.

Electroencephalographic (EEG) studies have delineated, with ever-increasing precision, the architecture of normal and abnormal sleep patterns. A unifying principle applicable to the nervous system is that every neuron in the nervous system finds connections to every other neuron.

As an example, a stimulus applied to the skin, such as a vibration, will travel through nervous system networks, eventually impacting upon and resonating into all cortical and subcortical structures, including the sleep centers.

The initial stages of sleep show characteristic brain wave configurations, namely slow waves and delta waves. Encephalographically-speaking, individuals afflicted with initial insomnia have difficulties in making the transition from waking brain wave patterns to patterns associated with the onset of normal sleep.

The method and apparatus may encourage this transition using vibrational prompting. In addition, auditory, visual, and electrophysiological stimulation contribute to impel the waking brain into adopting theta, and eventually, delta brain wave configurations.

Neurophysiological prompting is a process by which an external stimulus acts as an inducer for a desired physiological response. In this case, the desired response is the generation and the maintenance of delta sleep waves.

In order to facilitate the transition from the waking state to sleep, vibrational prompting can be synchronized to theta and delta wave frequencies. This vibrational prompting may be supplemented or supplanted by auditory, visual, and subliminal or para-subliminal electro-physiological stimulation.

The ability to perceive vibrational stimulation is called pallesthesia. Receptors in the skin and deeper tissues, including Pacinian corpuscules, relay their messages to the dorsal columns of the spinal cord, making their way to the thalamus and from there to somesthetic cortical areas for detailed recognition.

Along this trajectory, communications are made with multiple areas of the brain, including deeper structures. These include the hypothalamic sleep centers. In addition, once having reached the somesthetic cortex, vibrational impulses freely extend their reach into other cortical areas including the frontal, temporal, and occipital lobes. For example, it is appreciated that vibrations applied to the skin may be perceived visually.

Synesthesia is the phenomenon which describes such cross-sensory perception. The significance of this cross-sensory phenomenon is that, for example, a vibrational stimulus applied anywhere on the body will, given adequate time and repeated applications, create neural reverberations into many areas of the nervous system. If this vibrational stimulus is given a delta frequency, the effect will eventually make its way into brain sleep centers, which will be prompted to mimic this sleep-inducing rhythm.

The apparatus generates a desired brain wave frequency through a microprocessor unit. The frequency may be selected by the user, or may be predetermined. Thus, the unit frequency setting may be set anywhere from 8 to ½ cycles per second. Some individuals find that inducing theta waves (8 to 4 cycles per second) automatically paves the way for delta wave production. Others will prompt delta waves from the start.

Other options are possible. The unit, for example, may emit a sequential progression of frequencies which mimic the transition from the waking state (descending from 12 to 8 cycles per second), to stage 2 sleep (descending from 8 to 4 cycles per second), then on to delta sleep, from 4 to ½ cycles per second. Each of these stages may be programmed as to their respective durations.

The frequencies generated by the microprocessor are capable of driving different modalities of stimuli, either individually, or in combination. Among them:
1. Vibration. The device is capable of driving vibrational rhythms ranging from 8 to 4 cycles per second, thus mimicking Stage 1 sleep, and from 4 to ½ cycles per second, mimicking delta sleep. Vibrations generated by the vibrator unit and imparted to the sleep pad are directly transmitted to the individual via body contact.
2. Sound. The rhythm frequency may, in addition to vibrations, drive any one of a number of sounds, or tones. A menu of pleasing sounds may be chosen such as waterfalls, waves, musical instruments, or electronically generated sounds.
3. Colored lights. The rhythm frequency may drive colored lights as well. An LED (light emitting diode) or other light source, capable of being perceived by the sleeper through closed eyelids is incorporated in the device. Color preferences may be selected. A random presentation of colors may also be selected.
4. **Subliminal and para-subliminal electrophysiological stimulation.** The rhythm may also drive an electrophysiological stimulation unit (ESU). This ESU may emit microcurrents below the threshold of perception, or ones barely perceptible so as not to be distracting. These currents find their way into the autonomic nervous system, ultimately influencing the brain's sleep centers.

Thus, an apparatus and method can assist in the transition from wakefulness to sleep by means of theta and/or delta brain wave rhythmic prompting via vibrational stimulation applied to the skin. The apparatus is capable of supplementing or supplanting the vibrational stimulation with visual, auditory, and electrophysiological stimulation.

According to a preferred embodiment, the apparatus may comprise:
A. A flat pad of various dimensions or shapes. The illustration proposes a round pad with a diameter of 8 inches, which may or may not be made of electroconductive material, and which may show a picture suggestive of sleep. Many sizes, shapes, and designs are possible. The thickness of the pad is such that it will be comfortable to appose one's head to it. However, the user may also elect to appose the pad to other parts of the body.
B. A battery or other power supply which powers the various functions of the pad.
C. The pad incorporates a microprocessor which regulates several functions. The most fundamental function is the rhythm function which ranges from 12 to ½ cycles per second. However, the target rhythms range anywhere from 8 to ½ cycles per second, corresponding to theta (8 to 4 cps) and to delta (4 to ½ cps) brain wave rhythms. A rhythm may be pre-set, or may be assigned a sequential progression from waking brain wave rhythms (alpha, 12 to 8 cycles per second), to theta (8 to 4 cycles per second), and on to delta (4 to ½ cycles per second).
D. A digital readout and a rhythm light display showing the rhythm selected.
E. A vibrational motor unit capable of transmitting the selected rhythms to the pad so that the entire pad vibrates. The direction, intensity and frequency of the vibrations may be selected by the user.
F. A mini-speaker capable of translating the rhythm into sound. Modalities of sound may be selected to manifest tones, pleasing sounds of nature such as water flowing or waves, music, or electronically generated sounds. A volume control is included.
G. A light source capable of synchronous pulsations with the rhythm. The illustration shows a circular LED light source surrounding the pad. It is strong enough so that the user will perceive it with the eyes closed. Different colors may be selected.
H. An electrophysiological unit capable of imparting subliminal and para-subliminal electrical impulses to the pad. The unit emits microcurrent impulses to the pad in synchrony with the chosen rhythm, and determines its output power. Current, measured in milliamperes (mA), may range from 1 to 100 mA. Electrical pulse width may range from 5 to 500 microseconds (µs). The pulse rate may range from 1 to 2 pulses per second (pps) to 250 pps. An electroconductive solution may be applied to the pad so that electrical impulses may be more reliably imparted to the user.
I. A timer function capable of shutting off the unit after a selected time span; or capable of re-starting the unit at selected times during the night.

A preferred embodiment of a method may provide:
A. A process by which sleep is induced via the neurophysiological prompting of theta and, more importantly, delta brain waves. The latter brain waves are associated with Stages 3 and 4 NREM deep sleep.
B. The prompting is initiated by the presentation of one or more rhythmic stimuli which, singly or in unison, are capable of initiating nervous system resonance. Stimuli include vibration, light and color, sound, and electrophysiological stimulation.
C. An option to present the individual with a constant pre-selected rhythm within the span of the theta/delta range, namely 8 to ½ cycles per second.
D. An option to present the individual with a progression of rhythms designed to mimic the normal sleep pattern, namely a progression from alpha, to theta, and finally to delta rhythms.
E. An option to select the vibrational modality only, the light and color modality only, the sound modality only, the electrophysiological modality only, and any combination thereof.
F. An option to activate the pad at different times during the night.
G. An option to have an automatic shut-off.

**The anatomy and physiology of sensory mechanoreceptors.** Physiological factors are useful to consider in determining the therapeutic functions to be provided according to the method and apparatus.

The skin generates a constant flow of information, sending it to the spinal cord and the central nervous system for instantaneous processing and response. This information is essential for the organism's function and survival. Sensing the shape and feel of objects, their temperature, their movements and their possible injurious nature requires skin sensors that quickly translate many forms of mechanical energy in the environment into neurological signals.

Skin sensors are microorgans that inform on texture, pressure, heat, cold, vibration, and, importantly, on trauma and inflammation. For the latter, they generate pain signals.

Beyond skin, in deeper connective tissues of muscles, tendons and joints, other microorgans (e.g., spindles) also convey neurological information on body position and motion.

**Sensors in skin and connective tissues.**

Several types of skin sensors convey specific neurological information to the spinal cord and the central nervous system. Many receptors, however, while specialized, show some degree of cross-function. A touch sensor, for example, given a certain stimulus may, in addition to producing a signal of touch, also generate feelings of pressure.

The speed of nerve transmission from skin sensors to the spinal cord, and eventually to the brain, depends on the diameter of conducting nerve fibers, and on the degree to which they are sheathed in myelin, an insulating complex lipid.

The highly myelinated A fibers are large neuronal cables with conduction velocities of 70 to 120 meters/second. They carry sensation of proprioception, touch and pressure. C fibers, on the other hand, thin and unmyelinated, have conduction velocities approximating 1 meter/second. They carry pain sensations.

Sensory fibers with various conduction velocities conveying qualitatively different messages converge to the dorsal columns of the spinal cord, where they ascend to the medulla oblongata, the pons, the midbrain, and on to the thalamus, an ancient evolutionary brain structure. There, raw sensations gain conscious perception. Thalamic projections forward data to the post central gyrus in the cortex, where sensations are distilled into their finer subtleties via association networks. By way of cortical connections to the speech areas, for example, pain may be described as burning, aching, throbbing, dull, sharp or lancinating; and vibrations may be perceived as soothing, relaxing, or annoying.

**Sensors in the human skin.**

The skin transmits to the brain a variety of signals that give information on touch, vibration, cold, heat, pressure, and injury (which may be mechanical, chemical or electrical).

All skin and deeper tissue sensors convert stimuli in the environment into nerve impulses, which are basically electrochemical signals. How stimuli are presented (e.g., intensity, duration, quality) and perceived, forms the science of Psychophysics. How these electrical stimuli are eventually converted into the dimension of consciousness, for conscious perception, is unknown.

To achieve the task of translating a complex and fast-changing environment into sensory language, specialized tissue sensors have been developed. These sensors are fairly specific (e.g. light touch is only perceived as light touch and not as cold). However, given greater stimulation (e.g. such as pressure, added to vibration), many more receptors will be stimulated to fire and signals will thus have more representation and input impact in the brain.

The signals from the skin receptors course through the spinal cord to reach the posteroventral nucleus of the thalamus. From there, they are projected, via the thalamo-cortical projections, to the 6 layers of the post-central gyrus of the parietal lobe of the cortex, where they are given elaborate conscious meaning (e.g., differentiating the touch of velour from the touch of silk).

The central nervous system is completely interconnected via direct, circuitous, and feedback connections. In the thalamus, for example, the posteroventral nucleus of the thalamus has connections to all other thalamic and other nuclei, including the suprachiasmatic nucleus, involved in circadian rhythms and sleep. In other words, (pleasant) stimulation to the posteroventral nucleus will, especially if persistently continuous and rhythmical - for example at a rhythm of 1 to 8 Hz - make their way to the suprachiasmatic nucleus. The neurons in that nucleus will be prompted by these signals to generate neural signals in synchrony with them, sending signals having relevance to sleep to many regions of the brain. The signals it sends to the cortex will translate into delta and theta brain waves.

The above phenomenon invokes principles of neural synchronicity (where rhythmic signals have added power to involve more and more neurons), recruitment and neural entrainment, where neurons are increasingly coaxed into participation.

In this method and apparatus, optimal and pleasant vibrational stimuli are proposed to stimulate the posteroventral nucleus. With the addition of skin pressure, these stimuli come to have greater impetus.

Skin sensors work by mechanical deformation of their membranes. This deformation causes ion channels to open, thus creating depolarization and impulse discharge.

Several types of sensors found in the human skin and in deeper tissues provide a remarkable array of instantaneous information about many of the environment's variegated features:
1. Pacinian corpuscules, found in the dermis, are large by sensor standards and visible to the naked eye. Histologically, they appear as onion-configured concentric lamellae of connective tissue housing unmyelinated nerve roots. The friction of rubbing a finger on a textured object will induce vibratory stimuli registered by Pacinian corpuscules. Their fast adaptation makes them ideal for registering transient touch. Endowed with a large receptive field on the skin surface, they are sensitive to a range of vibrations of 60 to 400 Hz, or down to about 15 Hz with applied pressure, and with an optimal response at approximately 250 Hz.
2. Meissner's corpuscules are encapsulated dermal skin sensors endowed with unmyelinated nerve roots whose adaptive capacities make them responsive to vibrations of about 4-100Hz, and optimally to about 20-50 Hz.
3. Merkel's discs respond to minuscule distortions of tissues. Uncapsulated, unmyelinated and extremely sensitive, they are capable of the kind of tactile high resolutions needed in Braille. Their optimal vibrational responsiveness ranges between 2 and 30Hz, and optimally between 5 and 15 Hz.
4. Krause's bulbs are minute cylindrical bodies found in superficial skin layers and mucosal tissues. They respond to cold and to low frequency vibrations.
5. Ruffini cylinders are capsulated spindle-shaped receptors found in deeper skin layers. Heat and low frequency vibrations stimulate them.
6. Free nerve endings are unmyelinated neurons abundantly found in the epidermis that transmit signals possibly interpreted as pressure, warmth or pain in any one of its many variations. At low frequency, the signals may be interpreted pleasurably.
7. Warmth receptors differ from heat, coolness and cold receptors in that they are stimulated optimally at temperatures around 45° C. A warmth function may be provided as another avenue for skin stimulation. Provision is made for generating temperatures of 34° C to 47 C.

**Optimal mechanisms for the stimulation of skin and deeper structures.**

An intrinsic component of vibrational stimuli is pressure, which allows vibrations to displace tissues. In order to be maximally effective for a particular clinical task, vibration and pressure are adjusted to interface with the dynamics of skin receptor function, and with the mechanical properties of skin tissues, such as firmness and elasticity. At a pressure of 50 g/cm², for example, only a limited number of receptors will be stimulated. At a pressure of 1 kg/cm², many more receptors will be activated. The device preferably has a capacity to apply pressures to skin ranging from 25 g/cm² to 1 kg/cm² while generating vibrations.

Vibration and pressure can be adjusted separately. At a given vibratory rate, for example, light pressure will impart vibrations to superficial tissues only. At greater pressures, vibrations will impact deeper tissue depths.

Lateral displacement of vibrational stimuli, or vibrational amplitude, is important for stimulating skin receptors that are far apart. The minimal amplitude, corresponding to two-point skin discrimination is about 1mm. The device is preferably capable of generating vibrations with amplitudes ranging from 1 mm to 1.5 cm.

Irregular surface features on the device may augment the effectiveness of the stimulus. Indentations or corrugations may be linear, wavy, or circular, with a depth for example of 5 mm and a separation of about 1 mm to 1 cm.

Neuronal recruitment and sensory loading describe situations where the transmission of one modality, such as vibration, is assisted and even augmented by the stimulation of other receptors that use adjacent pathways to the spinal cord and brain (e.g., pressure, heat). The selective loading of sensory networks will produce increased traffic to the brain, involving more of its circuits, thus enhancing the influence of stimuli.

In view of the foregoing considerations, the disclosed method and apparatus provide vibrational stimuli configured to elicit selected mechanoreceptor skin responses in subjects. In addition, provision is made in the portion of the device that actually apposes itself to the skin surface of the subject, the interface. This interface, aside from the main vibrational stimulus it imparts (e.g., ½ to 8 Hz), may also impart subliminal and other vibrations of other frequencies concomitantly (8 to 250 Hz), in order to enhance neural recruitment. Additionally, the interface may also transmit heat to recruit warmth sensors; and it may be made of material whose surface is designed to stimulate touch and texture receptors in optimal fashion.

Thus, the method and apparatus create vibrational stimuli that are adapted to the physiology and neurology of the human skin; and to the properties of the nervous system, namely neural recruitment and sensory loading.

Different receptors may respond best to horizontal, vertical, or circular motions. This is due to their anatomy and their orientation in the skin. Vibrational stimuli, therefore, are best provided so that they can involve all these planes. Horizontal circular motions may be made to possess a vertical component so that vibrational stimuli may adopt a smooth repetitive 3-dimensional oscillation.

1. Vibrational stimuli are imparted to tissues with varying degrees of force. The resting pressure delivered by this device is self-adjusting according to demand. This is achieved via a microprocessor. At lower settings, the pressure is stimulating to a limited number of mechanoreceptors, mostly located in skin's surface layers. At higher pressure settings, it exerts influence on deep receptors in the dermal skin layers, and progressively deeper into connective tissues, muscles and joints. Light pressures, as in the first case, may be as low as 1 g/cm²; while in the second case, pressure may reach 1 kg/cm².

2. Lateral forces are important in vibrations that have horizontal movements. Pressure is applied to the vibratory motion in order to displace tissues laterally. Lateral movements stimulate receptors containing mechanoreceptors sensitive to sideways motions. The vibrations' horizontal displacement is believed to be optimal for human skin spans from 1 mm to 15 mm.

3. The device is capable of generating vertical pulsed stimuli. This allows for the stimulation of mechanoreceptors whose nerve roots respond to vertical compression. Predicated upon pressure gradients, vertical receptor stimulation may involve only superficial epidermal layers; or they may progressively reach deeper dermal tissues, supporting connective tissues, muscles, tendons and joints. The amplitude of these vertical vibrations is one factor in determining the depth of stimulation. The amplitude displacement of these vertical pulses optimal for human skin may span from 0.5 mm to reach the most superficial receptors, to 20 mm to involve deeper ones.

4. The device is capable of circular motions, which offers mechanoreceptor stimulation in horizontal planes, in all directions.

5. The device is capable of smooth repetitive 3-dimensional movements, which may include without limitation sinusoidal movements, which create mechanoreceptor stimulation in multiple vertical and horizontal planes.

6. Neural recruiting: the device, in order to augment the strength of its dominant vibrational signal (preferably ½ to 8 Hz), is capable of concomitantly emitting subdominant - and subliminal - vibration frequencies (8 to 250 Hz).

7. The interface element of the device that is in contact with the skin may be equipped with a heating element. Heat mechanoreceptors are thus stimulated, and sensory loading and neural recruitment are heightened.

8. The interface element of the device may be made of a material or have a configuration that maximizes touch receptor stimulation. Sensory loading and neural recruitment are thus augmented. Surface configurations, for example, may exhibit ridges, bumps or microprotuberances.

Other features and advantages of the disclosed method and apparatus will become apparent from the following description of embodiments, which refers to the accompanying drawings.

### BRIEF DESCRIPTION OF THE DRAWINGS

Fig. 1 is a top view of a sleep pad according to an embodiment.

Fig. 2 is a side view of the sleep pad.

Fig. 3 is a top view of the sleep pad with its top surface removed.

Fig. 4 is a side view of another embodiment of a sleep pad.

### DETAILED DESCRIPTION OF EMBODIMENTS

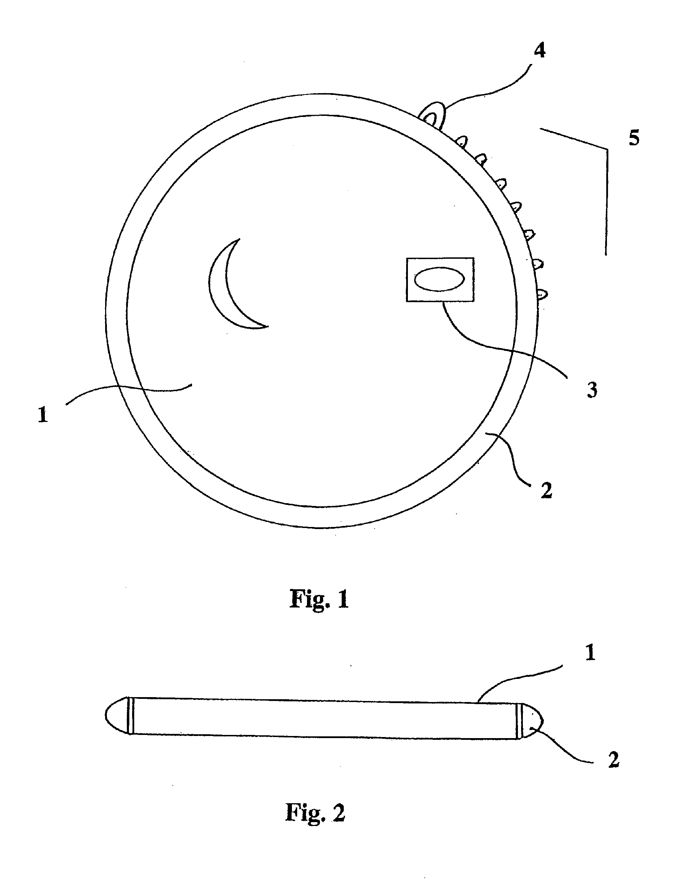

Fig. 1 shows the sleep pad in its frontal view. It shows a circular device approximately 8 inches in diameter, optionally adorned with a design suggestive of sleep on its surface (1). The other visible component is an LED array (2) around the rim, an LCD (liquid crystal display) (3), a rhythm light display (4), and programming buttons (5).

Fig. 2 shows a lateral view of the sleep device with the surface element (1) and the LED (light emitting diode) array (2) on the outer edge.

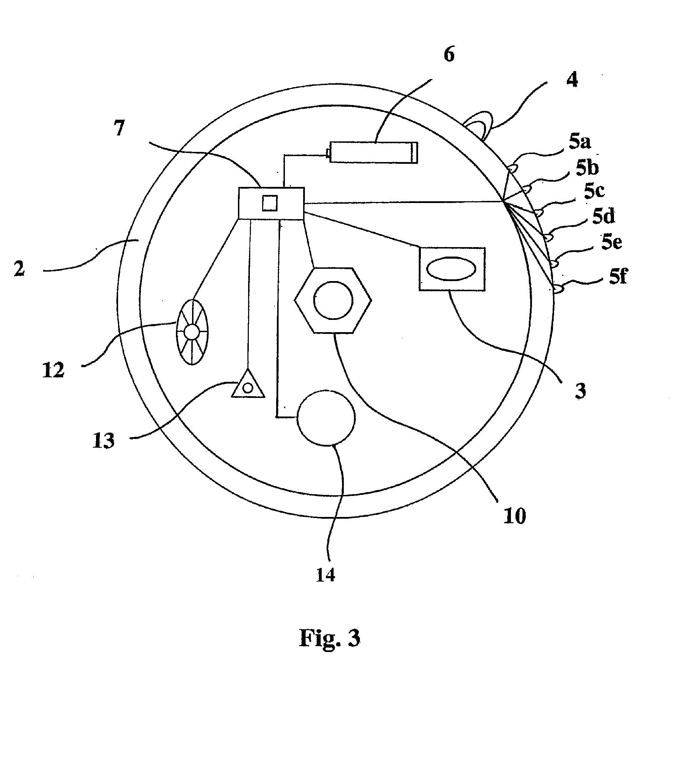

Fig. 3 shows the sleep pad without the surface element. Shown schematically therein are the working elements of the sleep device including the battery (6) which energizes the microprocessor (7). The microprocessor is programmable via the control buttons (5a), (5b), (5c), (5d), (5e), and (5f). Button (5a) programs the rhythm function which is both displayed on the LCD (liquid crystal display) (3), and the rhythm light display (4). Button (5b) programs the motor unit (10). Button (5c) programs the speaker (12). Button (5d) programs the LED (light emitting diode) array (2). Button (5e) programs the electrophysiological unit (13). Button (5f) programs the timing function of the microprocessor (7).

The programmable microprocessor has a variety of functions. Foremost is the rhythm function programmed through control (5a). The rhythm selection is shown on the LCD display (3). The rhythm control may select a fixed rhythm, or may select a sequence of rhythms, such as a rhythm progression from alpha (12 to 8 cycles per second), to theta (8 to 4 cycles per second), on to delta (4 to ½ cycles per second), for example.

As indicated above, frequency ranges of ½ to 8Hz and preferably including 8-250Hz are provided. The motor unit (10) is preferably capable under control of the microprocessor (7) of horizontal, vertical, circular and 3-dimensional movements, or at least some of these in combination.

The respective rhythms may be generated for variable corresponding lengths of time. The timer control (5f) programs the desired time parameters of the unit including automatic shut off and re-start.

The apparatus presents as a flat pad made of pliable and electroconductive material, such as, for example, carbon silicone. It is thin and comfortable enough to rest one's head upon it. Yet, it may be apposed to any part of the body. In the illustration in Fig. 1, this pad is 8 inches in diameter and is adorned with a design suggestive of sleep. The pad, however, may adopt any suitable size, configuration, and design.

The functions of the sleep pad of greatest therapeutic value are believed to be the vibrational capacity of the motor unit (10), and the warmth-generating heater (14). However, in addition, it has the capacity, predicated upon individual choice or therapeutic preference, to express rhythmic light, rhythmic sound, and rhythmic electrophysiological stimuli.

The sleep pad is provided with an energy source, a battery, Fig. 3 (6) This battery powers a microprocessor, Fig. 3 (7), which regulates all the functions of the device, among them:
A. The motor unit, Fig. 3 (10).
B. An LED light display, Fig. 3 (2).
C. A sound source, Fig. 3 (12).
D. An electrophysiological stimulation unit, Fig. 3 (13).
E. A rhythm light display, Fig. 3 (4).
F. A timer.
G. The heater (14).

The microprocessor may be programmed to:
A. Emit a set rhythm, with a choice of frequency from ½ to 8 and 8-250 cycles per second.
B. Emit any one of several rhythms in progression.
C. Sustain a rhythm for a time period ranging from less than a minute to more than an hour through the use of a timer.
D. Activate the rhythm at various times during the night.
E. Activate the vibrational unit only, so that the entire pad vibrates in synchrony with the chosen rhythm.
F. Activate the light source only, to pulse with the chosen rhythm.
G. Choose the intensity and the color of the light source.
H. Activate the sound function only, in synchrony with the rhythm, and regulate its volume.
I. Choose the type of sound emitted: tone, nature sounds, music, electronically synthesized sounds.
J. Activate the electrophysiological unit only, to emit bursts of microcurrent pulses to the pad in synchrony with the chosen rhythm, and to determine its output power. The microcurrents may be subliminal, below the level of awareness of the user, or may be para-subliminal. The duration of the bursts may be constant or variable and may be selected according to the user's preference or for therapeutic reasons. Current, measured in milliamperes (mA), may range from 1 to 100 mA. Electrical pulse width may range from 5 to 500 microseconds (µs). The pulse frequency may range from 1 or 2 pulses per second (pps) to 250 pps.
K. Activate any combination of the above modalities, including all of them, in unison.

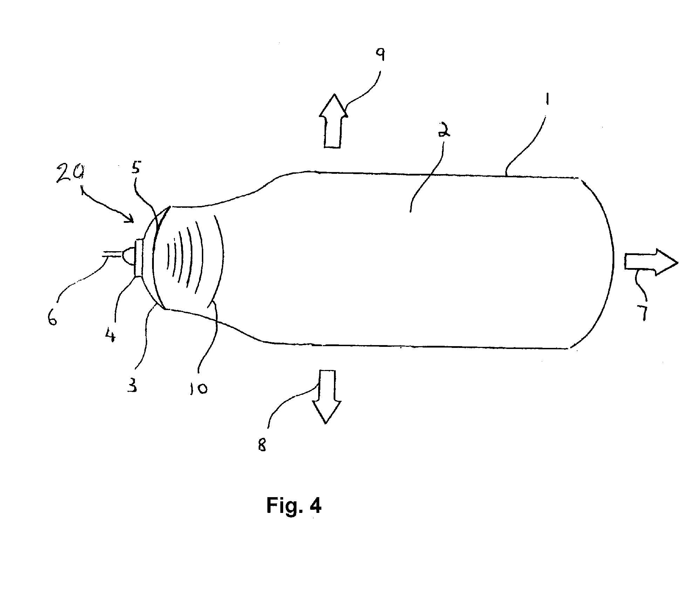

In a preferred embodiment, shown in Fig. 4, the motor unit employs a speaker or speaker-like electromagnetic vibrator 20 which provides vibrations, as already described herein in connection with the other embodiments. The vibrator 20 is controlled to vibrate between about 1Hz and 8Hz, with 10 preset amplitude settings.

In the embodiment of Fig. 4, an envelope 1 is made of plastic, silicone, or any other pliable material capable of being hermetically sealed. Contained within the envelope 1 is a medium which may be a gas, such as air, or a fluid or a gel, such as a silicone gel. The vibrator has a vibration generator 4 which works on electromagnetic principles, and drives a cone-shaped transducer 3 which transmits vibrations to the medium 2 at an interface 5. The vibrator may be a low-frequency speaker. Power and control signals are supplied at an input 6. Vibrations 10 are generated in the fluid, gel, or gas medium. The vibrator generates both lateral vibrations 8, 9 and longitudinal vibrations 7 in the medium 2. The envelope 1 is hermetically sealed and the interface 5 abuts the outside of the envelope 1. However, in another embodiment, the transducer 3 could be hermetically sealed inside the envelope 1, or form a part of the envelope 1.

Preferably, a suitable heater (not shown) may be provided, for maintaining the medium 2 at a comfortable temperature, as already described.

The embodiment of Fig. 4 may be contained in a casing such as that shown in Fig. 2, or may be placed in direct contact with the user in the manner of a pillow or hot-water bottle. The size and shape are selectable in accordance with the treatment described for the user.

Although embodiments have been described in some detail, it will be understood by those skilled in the art that changes and modifications may be practiced without departing from the spirit and scope thereof.

## Claims

1. An apparatus for generating stimuli for prompting the production of slow brain waves and thereby inducing sleep, comprising in combination:
a programmable controller operable for prompting the production of slow brain waves by setting and generating a sleep-inducing rhythm; and
a transducer unit containing at least one motor unit connected to said controller for receiving said rhythm and generating and applying at least one of horizontal, vertical, circular and smooth repetitive 3-dimensional vibrations as said stimuli to prompt the production of slow brain waves of a user in accordance with said rhythm.

2. The apparatus of claim 1, wherein said motor unit generates at least two of said horizontal, vertical, circular, and smooth repetitive 3-dimensional vibrational stimuli.

3. The apparatus of claim 2, wherein said motor unit generates pressure in a range of 1 g/cm² to 1kg/cm².

4. The apparatus of claim 2, wherein said motor unit generates horizontal movement of 1 mm to 15 mm.

5. The apparatus of claim 2, wherein said motor unit generates vertical movement of 0.5 to 20 mm.

6. The apparatus of claim 1, wherein said transducer unit further comprises a heater for applying a temperature of approximately 34°C to 47°C.

7. The apparatus of claim 1, wherein the transducer unit further has irregular surface features having a depth of up to about 5 mm and a separation of about 1 mm to 1 cm.

8. A method of prompting the production of slow brain waves and thereby inducing sleep in a user, comprising the steps of:
juxtaposing a transducer unit with said user;
using a programmable controller to program a sleep-inducing rhythm;
supplying said rhythm from said controller to a motor unit in said transducer unit and thereby generating and applying at least one of horizontal, vertical, circular, and smooth repetitive 3-dimensional vibration as stimuli to prompt the production of slow brain waves of said user.

9. The method of claim 8, wherein said motor unit generates at least two of horizontal, vertical, circular, and smooth repetitive 3-dimensional vibrational stimuli.

10. The method of claim 9, wherein said motor unit generates pressure in a range of 1g/cm² to 1kg/cm².

11. The method of claim 9, wherein said motor unit generates horizontal movement of 1 mm to 15 mm.

12. The method of claim 9, wherein said motor unit generates vertical movement of 0.5 to 20 mm.

13. The method of claim 8, wherein said transducer unit further comprises a heater for applying a temperature of approximately 34°C to 47°C.

14. The method of claim 8, wherein the transducer unit further has irregular surface features having a depth of up to about 5 mm and a separation of about 1 mm to 1 cm.
